# EUROPEAN PATENT APPLICATION

(11) **EP 3 318 294 A1**
(43) Date of publication of application: **09.05.2018**
(21) Application number: 16306468.6
(22) Date of filing: 08.11.2016
(51) Int. Cl.: A61M 5/14, A61M 39/02

(54) **KIT FOR IN SITU DELIVERY OF A COMPOUND OF INTEREST**

(71) Applicant: Defymed, 67200 Strasbourg (FR)
(72) Inventor: SIGRIST, Séverine, 67200 STRASBOURG (FR); BOU AOUN, Richard, 67200 STRASBOURG (FR); BURCEZ, Charles-Thibault, 67200 STRASBOURG (FR)
(74) Representative: Flesselles, Bruno F.G.

(57) **Abstract**

The invention relates to a kit for delivering a compound of interest *in situ* to a patient in need thereof, comprising a chamber (2) comprising a closed shell made of the semi-permeable membrane, the pouch comprising at least one connector (3) comprising a body attached to the sheet, and a catheter (4) that can be connected to said connector at one end and that can be connected to a source of delivery of a compound of interest at the other end, as well as methods of use of such kit.

## Description

The invention relates to a kit for delivering a compound of interest *in situ* to a patient in need thereof, and further describes methods of use of such kit.

There is a need to deliver compounds of interest in vivo to patients in need thereof. For some diseases and conditions, such compound of interest needs to be provided to the patient on a regular basis (whether continuously or not).

In particular, in type I and some Type 2 diabetes mellitus, the patient will need to have an insulin injection close to the meals or when there is food intake in order to maintain as constant as possible the glycaemia level.

There are multiple ways to inject a compound of interest and lots of them are used for injecting insulin to a patient in need thereof.

In particular, a patient can use insulin syringes or insulin pens (with direct subcutaneous insulin injection). In the past few years, other devices have been developed, in order to deliver insulin in a more proper *in situ* place that would be more relevant from a physiological point of view. Consequently, insulin pumps (external or implantable) have been developed, that would deliver insulin to the patient's body through a catheter that is implanted subcutaneously for external pumps. One can cite the Accu-Chek® DiaPort system developed by Roche, which is intended to enable the infusion of insulin into the peritoneal cavity using an insulin pump and a cannula infusion set, with a catheter placed in the abdomen. The delivery is also intraperitoneal when using implantable pumps.

However, there are some problems associated with these devices. In particular, there exists a risk of clog at the end of the catheter at the location of insulin delivery when it is delivered *in situ* (as opposed to when delivered in the fat subcutaneously). Furthermore, there may be some fibrin deposit around the end of the catheter, in particular due to scar tissue developed at the site where the cannula is inserted, that requires the patient to regularly inspect the pump (at least once a day) to see if an "Obstruction" message is displayed and change the catheter when this is the case because plugging of the catheter would prevent insulin to be distributed and immediately raise blood sugar.

An external insulin pump indeed delivers insulin by pushing it through a catheter connected to a small subcutaneous cannula (soft or solid (made from steel) cannula) which needs to be changed every two or three days. Due to the frequency of changing the catheter, this may result is the skin being irritated due to the adhesive securing the cannula, or having some difficulties to heal. This also requires some hygiene precautions at the implantation site.

The delivery systems, whether in particular with subcutaneous implantation, may also prove to be limited in patient with severe hypoglycemia, due to the lack of hepatic extraction of insulin with this mode of administration.

There may also be an infectious risk with the devices of the prior art such as the Diaport®, as there is a direct access from the outside of the body to the intraperitoneal site, which is a sensitive site. One can observe dermo-hypodermic infections around the device, local and recurrent infections, and iterative occlusions. Clinical trials have shown that multiple complications arose with this device, despite an improvement in the glycaemia balance. The long-term risk-benefit balance is thus likely not very favorable.

Herein is provided a kit and system to be able to deliver a compound of interest, and in particular insulin at a site of interest within the patient's body. This compound of interest is not delivered intravenously, but near an organ of interest.

The compound of interest is delivered through a chamber delimited by at least one semi-permeable membrane, with a catheter arriving to the chamber and delivering the compound of interest within the chamber, the compound of interest being able to diffuse through the semi-permeable membrane to the patient's body.

As compared to the Diaport® system, the device herein proposed is a closed system, which makes it possible to deliver insulin (or another compound of interest) at a site *in situ* (such as an intraperitoneal or extraperitoneal site) through the implantable chamber rather than directly at the exit of the catheter. This limits the communication between the site of delivery and the outside, and the infections are lowered, in particular when an injection port (in particular sub-cutaneous) is further part of the kit and used.

The semi-permeable membranes are permeable to compounds of interest, but not to cells or platelets.

The chamber is impermeable to the cells of the patient and will be vascularized after implantation (especially if it is covered with an appropriate molecule), thus leading to efficient release of the compound of interest within the patient's circulation. Furthermore, due to the surface of the chamber, the release of the compound of interest is not at a specific point, but more diffuse, thus reducing the risk of clogging, or fibrin deposit. The chamber can be implanted at a site chosen by the physician, which can thus ensure that the delivery of the compound of interest is made at the most appropriate (in particular from a physiological point of view) location to optimize the biological effect of the compound of interest. This may allow to reduce the quantity of the compound of interest that is delivered to the patient (as the efficacy would be increased), and hence adverse effects.

### DESCRIPTION OF THE INVENTION

The invention relates in particular to a kit comprising
a) a chamber comprising a closed shell [pouch] made of the semi-permeable membrane, wherein the pouch comprises at least one connector comprising a body attached to the sheet, and a conduit connected to the connector so as to be in hydraulic communication with the inside of the pouch
b) at least a catheter that can be connected to said connector at one end and that can be connected to a source of delivery of a compound of interest at the other end.

In a particular embodiment, said catheter presents an injection port, in particular implantable subcutaneously, at the end that can be connected to a source of delivery. This embodiment is preferred as it makes it possible to deliver the compound of interest *in situ* within the patient's body, by making a simple subcutaneous injection and with a low risk of infection. Furthermore, it makes it possible to improve healing.

In a particular embodiment, the kit further comprises a vessel containing said compound of interest.

In a particular embodiment, the kit further comprises a pump making it possible to send the compound of interest from the vessel to the chamber within the catheter. This embodiment would make it possible to allow continuous injection of the compound of interest. In this embodiment, the system (chamber + catheter) could remain in place for more than 3 days after implantation on the patient (whereas the current systems generally need to be changed at this frequency), and this would thus limit the contamination risk, improve healing and improve patient's comfort.

In another embodiment, the kit further comprises a device for single shots of the compound of interest, such as an insulin pen/needle.

In a particular embodiment, the internal diameter of membranes pores is comprised between 5 nm and 2000 nm, preferably between 10 nm and 1200 nm, more preferably between 10 and 900 nm.

In a particular embodiment, the membrane of the chamber comprises multiple layers of biocompatible polymers, in particular at least one layer of porous biocompatible polymer, and one layer of non-woven biocompatible polymer.

In a particular embodiment, the membrane comprises, on its external surface, a biologically active molecule, in particular heparin, which can be covalently bound to a layer on the surface of said membrane.

In a particular embodiment, the kit further comprises sensors and/or captors to measure the level of a given biomarker in the blood and optionally send signals to the external source of the compound of interest.

In a particular embodiment, said biomarker is glucose and wherein said compound of interest is insulin.

In a particular embodiment, said source of delivery of the compound of interest is a syringe, an artificial bio-organ, an implantable or external vessel optionally also comprising a pump.

The invention also relates to a method for delivering a compound of interest to a patient comprising the steps of using the kit as described herein in order to deliver the compound of interest to the interior of the chamber, from the external source, through the catheter.

In a particular embodiment, the chamber has previously been implanted between the muscles (rectus or transversus abdominis muscles) and the peritoneum.

In a particular embodiment, the quantity and/or rate of the delivered compound is adjusted in response to the signal received from the sensors/captors if such are present.

Also part of the invention is a method of using the kit as described herein, comprising the step of surgically implanting the chamber between the muscles (rectus or transversus abdominis muscles) and the peritoneum, and optionally further comprising the step of attaching one end of the catheter to the connector of the implanted chamber and/or further comprising the step of attaching the other end of the catheter to a vessel, in particular through an injection port.

### DETAILED DESCRIPTION OF THE INVENTION

It is recalled that the term "biocompatible" is said of a material which is well tolerated by a living organism and which does not cause a rejection reaction, a toxic reaction, a lesion or a harmful effect on the biological functions of the latter. This does not exclude the possibility of an inflammatory reaction due to the insertion of the material into the organism or of an immune reaction in the case of a biocompatible organ comprising exogenous cells; this immune reaction is not therefore due to the organ as such, but instead due to its content (secretion of chemokines by the exogenous cells).

A membrane is semi-permeable when it presents a cut-off threshold, the molecules having a weight above this cut-off threshold being unable to cross the membrane, while the molecules having a weight below this cut-off threshold can cross the membrane. Such semi-permeability can be obtained with a porous membrane, with an appropriate size of pores. The determination of the cut-off threshold is carried out by those skilled in the art according to the characteristics of the molecules that they wish to stop or allow to penetrate. A semi-permeable membrane is thus a membrane that presents at least one layer of a porous polymer.

The internal diameter of the pores of the porous polymer makes it possible to obtain the desired cut-off threshold (which is to not allow platelets to enter within the chamber). Thus, in one particular case, the internal diameter of the pores present on the layer of porous biocompatible polymer is between 5 nm and 2000 nm, preferably between 10 nm and 2000 nm, in particular between 10 nm and 1200 nm. A size of pores between 10 nm and 800 nm, in particular between 10 nm and 600 nm is perfectly suitable.

WO 2015/086550 describes membranes comprising one layer of non-woven polyer and at least one layer of porous polymer. This application describes in particular membranes where the non-woven polymer is between two layers of porous polymer. It is specified that at least one of the two layers (or the only layer if such is the case) of porous biocompatible polymer has pores which have an internal diameter greater than 5 and preferably greater than 10 nm, and less than 100 nm, and preferably greater than 10 nm and less than 50 nm, more preferably less than 40 nm. When the membrane has two layers of porous biocompatible polymers, the internal diameter of the pores of one of the layers is preferentially as above. The internal diameter of the pores of the second layer may be the same or larger, the cut-off effect at the desired size being given by the diameter of the pores of the first layer. Thus, the internal diameter of the pores of the second layer may be greater than 100 and less than 2000 nm, preferably greater than 200 nm. These pores preferably have an internal diameter less than 1000 nm. An internal pore diameter greater than 400 and less than 600 nm, or of approximately 500 nm, is perfectly suitable. These intervals of diameters can be used in the context of the present invention.

### Description of the chamber

The invention uses a chamber for allowing *in situ* diffusion of at least one compound or substance of (therapeutic) interest. This chamber comprises a closed shell made of a functionalized membrane as described herein, delimiting a volume in which the compound of interest will transit from the catheter, and before *in situ* diffusion.

This encapsulating chamber can also be referred to as a "pouch". In one embodiment, the exterior (outer part) and interior (internal part) of the chamber is functionalized with a molecule having biological activity, such as heparin.

Such chambers are described in application WO 2012/010767. In one preferred embodiment, the shell is formed from two membranes as disclosed herein, which are heat-welded together. Use may be made of the method described in WO 2012/010767 or a method of heat-welding using ultrasound, known in the art. Membranes can also be sealed on their periphery, to form the chamber, using any thermosealing method known in the art.

### Shape of the chamber

In one preferred embodiment, the encapsulating is circular. Such a shape has several advantages:
- absence of "corners" or protruding parts which are capable of creating cell or inflammatory aggregates during the implantation,
- ease of manufacture of the encapsulating chamber In one particular embodiment, the diameter of the chamber is greater than 2 cm. It is generally less than 20 cm, and is preferentially less than 15 cm, or than 12 cm. A diameter of between 2 and 12 cm is perfectly acceptable.
- When the chamber is not round, the largest dimension thereof is generally greater than 2 cm. It is generally less than 20 cm, and is preferentially less than 15 cm, or than 12 cm. A largest dimension of between 2 and 12 cm is also perfectly acceptable.

### Volume of the chamber

As seen above, the chamber delimits a volume into which the solution containing the compound of interest will transit before diffusion at the exterior of the chamber, through the pores of the membrane forming the envelope of the chamber.

These external and internal layers may be functionalized with heparin or another molecule as disclosed below.

The encapsulating chamber comprises at least one connector (in particular attached to the shell), which makes it possible to establish a communication between the exterior and the interior of the shell and to connect the catheter of the kit.

One should note that it is possible to functionalize (such as bridging heparin) the membranes before forming the encapsulation chamber. In this case, as indicated elsewhere, the heparin should be, at least, on the outer surface of the chamber.

Alternatively, it is possible to form the chamber and then functionalize it, for instance by dipping it in various baths containing the priming solution, and the solution containing the functionalizing molecule.

### Description of the membrane forming the chamber

### Non-woven polymer

A non-woven polymer (non-woven) is such that the fibers thereof are maintained randomly. It is thus a sheet consisting of fibers oriented in a particular direction or randomly, bonded by friction and/or cohesion and/or adhesion. The fibers are thus arranged statistically, i.e. deposited randomly. Consequently and due to the random arrangement of the fibers, the non-woven polymer is permeable to substances, and there is no control of the size of the substances that can diffuse within the non-woven polymer. A non-woven membrane is not structured as the porous membranes and there is no control of diffusion of substance within the non-woven part of the membrane, in contrast to porous membranes, which present a cut-off, depending on the size of pores, as described below.

Non-woven polymers can be produced using polymeric fibers of any type. Mention may thus be made of polyesters: PET (poly(ethylene terephthalate)), PBT (poly(butylene terephthalate)), PVC (poly(vinyl chloride)), PP (polypropylene), PE (polyethylene) or blends of these polymers.

Polyamides or polycarbonates can also be used to produce non-woven polymers.

Preferably, the non-woven polymer is chosen from polycarbonate (PC), polyester, polyethyleneimine, polypropylene (PP), poly(ethylene terephthalate) (PET), poly(vinyl chloride) (PVC), polyamide and polyethylene (PE). Blends of these polymers can also be used for producing the non-woven polymer. Poly(ethylene terephthalate) (PET) is particularly preferred.

Generally, this non-woven polymer is obtained by the meltblown method. The composition thereof is an entanglement of microfibers which have been "melt blown". The fibers have been obtained by any method known in the art, in particular the polymers herein used can be obtained by any method known in the art, in particular electrospinning, which avoids use of solvents, which is advantageous for the clinical application of the device and kit.

This method of production is particularly suitable for polymers which can be melt spun, in particular polypropylene, poly(ethylene terephthalate), polyamides or polyethylene.

This method generates non-wovens of greater mechanical strength.

Usable materials are disclosed in particular in WO2015086550, PCT/EP2016/061051, EP1357896 or WO2012010767.

### Porous biocompatible polymer

A porous biocompatible polymer is known in the art. It may be chosen from polycarbonate (PC), polyester, polyethyleneimine, polypropylene (PP), poly(ethylene terephthalate) (PET), poly(vinyl chloride) (PVC), polyamide and polyethylene (PE). The polymers herein used can be obtained by any method known in the art, in particular electrospinning.

The use of a porous biopolymer in the chamber's membrane will make it semi-permeable.

In one particular embodiment, at least one layer of the membrane is made of poly(ethylene terephthalate) (PET).

The pore formation is carried out by any method known in the art. In particular, it is possible to use the electron bombardment method or the heavy ion bombardment method (this second technique is in particular described in patent US 4 956 219). In the case of heavy ion bombardment, the density of the heavy ions bombarded at the surface of the biocompatible support determines the pore density, while the chemical erosion treatment time determines the pore size.

The porous membranes are thus prepared using the "track-etching" process known in the prior art and described in particular in patents US 4 956 219, DE19536033 or CH701975.

This technology consists in irradiating a polymer film by means of energetic heavy ions, resulting in the formation of linear latent traces characterized by a local degradation of this polymer; these traces are then revealed in the form of pores by means of a selective chemical attack.

The membrane is beamed with heavy ions. The heavy ions pass through the entire thickness of the polymer film. In passing through the polymer, the heavy ions destroy or cut the polymer chains and thus form a clean straight opening through the material. The final alignment of the pores is determined by the angle of the beam relative to the polymer film during the irradiation process. The beam may thus be perpendicular to the polymer film or at any other angle.

In the next step, the film is passed through a bath of a strong acid such as nitric acid and the openings become pores after contact with alkaline solutions such as sodium hydroxide or potassium hydroxide.

Contrary to the rest of the film, these openings made by the ions allow the alkaline solution to pass through, said alkaline solution filling them and allowing the etching of the pores by removing the material (polymer) around these openings.

The pore size is controlled by the concentration of the alkaline solution, the contact time and the temperature of the solution.

If polyester or polycarbonate is used, the membrane obtained is hydrophilic and can either be used as it is or else be treated using surface treatment processes (plasma, spraying or coating).

The preparation of membranes according to this "track etching" technology is more precisely described in patents US 4 956 219 and CH701975.

This technology enables the production of porous polymer membranes characterized in particular by a flat surface and a narrow cut-off threshold.

The advantage of using membranes obtained by this technology is the great accuracy of the pore size, of the number of pores, and of the shape of the pores.

The pores are preferentially cylindrical, but this technology can also make it possible to obtain pores of other shape, such as of conical shape.

Preferentially, the pores are aligned, and have an angle of between 10° and 45°, relative to the vertical, but can also have angles > 45° or < 10°. These angles are obtained according to the angler of the beam of ions during the beaming of the membrane.

This technology is applicable to various materials, such as polycarbonate (PC), polyester (PET) or polyimide (PI). Polyamide, poly(vinylidene fluoride), polyacrylate or polyolefins can also be used.

This method makes it possible to easily obtain pores with a controlled size of between 5 nm and 2000 nm, a pore density of between 10³ pores/cm² and 10¹¹ pores/cm² and membranes with a thickness of between 5 µm and 80 µm.

It is to be noted that, without the treatment to form pores on the biocompatible polymer, such layer of biocompatible polymer would remain impervious to any substance, and would not allow diffusion of the substance of interest from the inner part of the biocompatible organ to the outer part. The pores only allow the diffusion of substances that are below the cutoff (*i.e.* that are smaller than the pore diameter). In this specific use, the most important is to avoid platelets aggregation within the chamber, and the cut-off will thus be chosen as to forbid entry of platelets in the chamber.

It is thus clear that the layer of the non-woven biocompatible polymer and layer of the porous biocompatible polymer are different layers, made of different materials, and presenting different properties (in particular with regards to the passing and diffusion of substances through each layer).

Materials are disclosed in particular in WO2015086550, PCT/EP2016/061051, EP1357896 or WO2012010767.

### Membrane forming the chamber

The membrane forming the chamber will comprise a porous layer of a biocompatible polymer, as disclosed above. This will make it semi-permeable, the exclusion size being correlated to the maximal size of the pores diameter.

This membrane forming the chamber may present only one layer of polymer, or comprise multiple layers.

In particular, the semi-permeable membrane, making the chamber and delimiting the volume through which the compound of interest will transit before *in situ* diffusion, may comprise at least one layer of porous biocompatible polymer, and one layer of non-woven biocompatible polymer, as disclosed in WO 2015/086550. All technical information provided in this application, related to the chamber, can be adapted to the chamber to be used in the kit herein described.

In a particular embodiment, the membrane comprises two layers of porous biocompatible polymers, on either side of a layer of biocompatible non-woven polymer. Thus, this layer of biocompatible non-woven polymer is located, positioned or situated between these two layers of porous biocompatible polymers.

Such an embodiment makes it possible to optimize the strength and resistance of the chamber. Indeed, this layer of non-woven can be considered to behave like a "sponge", which gives it the capacity to absorb impacts and to deform, thus increasing the rigidity and resistance of the membrane *in situ.* Locating the layer of non-woven between two porous layers of biocompatible polymers thus makes it possible to provide the device with additional protection/strength, and with no effect on the molecular diffusion of the biological substances.

It is not necessary for the porous and non-woven biopolymers to be identical.

Likewise, in the presence of two layers of porous biopolymers, the latter can be the same polymer or different polymers.

When the membrane presents two layers of porous biopolymers, it is possible to functionalize only one of them with a molecule with biological activity such as heparin. Alternatively, both layers of porous biopolymers are functionalized with such molecule.

### Other polymers

It is possible to add various layers of polymers (such as any hydrophilic polymer) to the surface of the membrane, and in particular on the surface of the porous polymer. These layers of polymer can be added by using the Layer-by-Layer deposition method. Examples are provided in WO 2012/017337.

A hydrophilic polymer is a polymer or a blend of polymers, which, after application on a film of porous biocompatible polymer, has an angle value of less than 40°, preferably less than 30°, after measurement according to the "sessile drop" test described in Example 2 of WO 02/060409.

It should be noted that the angle value according to the "sessile drop" test can vary depending on the treatment of the polymer. Thus, contact angles of less than 20° (of about 16-17°) can be observed for the biocompatible biopolymer, when two plasma treatments are carried out, this angle increasing (generally less than 30°) when the hydrophilic polymer (in particular HPMC) is deposited after the two plasma treatments. If a blend of hydrophilic polymers, which also contains a molecule with biological activity (in particular an HPMC, ethylcellulose + heparin mixture), is used, the angle may be greater than 30°, but remains less than 40°.

Preferentially, the hydrophilic polymer is soluble in water. This is because, due to of the implantation of the bioartificial organ in the body of a host organism, use of organic solvents is excluded since their complete elimination is difficult, and their presence, even in small amounts, is not compatible with a therapeutic or surgical use in humans or animals.

Preferably, the hydrophilic polymer material is chosen from the following hydrophilic polymers:
- celluloses and derivatives thereof, such as ethylcellulose (EC), hydroxypropylmethylcellulose (HPMC) or carboxymethylcellulose (CMC);
- polyacrylamides and copolymers thereof;
- polyvinylpyrrolidone (PVP) and copolymers thereof;
- polyvinyl alcohols;
- vinyl acetate copolymers, such as a poly(vinyl acetate)/poly(vinyl alcohol) copolymer;
- polyethylene glycols;
- propylene glycols;
- hydrophilic poly(meth)acrylates;
- polysaccharides;
- chitosans.

As hydrophilic polymer, use is made of both a polymer material consisting of one of the hydrophilic polymers as defined above and a blend of several of the hydrophilic polymers above, generally a blend of two or three of the hydrophilic polymers above.

Preferably, the hydrophilic polymer is chosen from cellulose-based compounds, in particular HPMC, EC, TEC or CMC, polyvinylpyrrolidones, poly(vinyl alcohol)s, or polyacrylates such as poly(hydroxyethyl acrylate) (HEA) or acrylic acid copolymers.

The hydrophilic polymer may also be composed of a blend of two or more hydrophilic polymers mentioned above, in particular a blend of HPMC and CMC, or of HPMC and EC.

Celluloses and cellulose derivatives, in particular hydroxypropylmethylcellulose (HPMC), are preferred.

### Membrane lamination

As indicated above, for greater mechanical stability, it is preferred when the porous biocompatible polymer membrane is reinforced with a layer of a non-woven polymer.

The combination of a layer of a non-woven polymer and of a layer of a porous biocompatible polymer is preferentially carried out by lamination, using methods known in the art, such as thermal lamination, with or without the presence of adhesives, preferably without adhesive.

Thus, the reinforcement of the membrane can be improved via a multilayer system alternating layers of woven or non-woven polymers and of biocompatible porous polymers. However, any degradation of the diffusion properties should be avoided.

In particular, the mechanical stability can be increased by combining a thin functional membrane which has a high pore density with a thick protective membrane which has a low pore density.

There is no limitation to the number of layers of polymers that can be used to manufacture the membrane.

It is also possible to perform a surface treatment of one or more of the polymer layer (porous polymer and/or non-woven polymer) such as plasma or corona treatment.

One can to see the different individual and distinct layers and that the non-woven and porous layers are not identical or confused one with the other by Electron Microscopy of the membrane.

### Addition of an active molecule at the surface of the membrane

It is possible that a hydrophilic polymer deposited on the layer of porous biocompatible polymer can optionally contain an active molecule.

This "active molecule" is mixed with the hydrophilic polymer. It is intended to be released into the medium surrounding the semi-permeable membrane in particular in order to reduce the inflammation due to the implantation of the bioartificial organ, and/or to induce a positive response (in particular increased vascularization) by the tissue(s) of the patient receiving the bioartificial organ.

Thus, the active molecule is chosen from anti-inflammatory agents, anti-infective agents, anaesthetics, growth factors, agents which stimulate angiogenesis and/or which induce vascularization, agents which induce healing, immunosuppressive agents, antithrombotics including antiaggregants and anticoagulants, angiotensin-converting enzyme (ACE) inhibitors, or any molecule which stimulates insulin secretion (IGF, glucagon-like peptide 1 (GLP-1) or its derivatives, incretin mimetics).

Among the anti-inflammatory agents, mention may be made of nonsteroidal anti-inflammatories (NSAIDs), such as acetaminophen, aminosalicylic acid, aspirin, celecoxib, choline magnesium trisalicylate, declofenac, diflunisal, etodolac, flurbiprofen, ibuprofen, indomethacin, interleukin IL-10, IL-6 mutein, anti-IL-6, NO synthase inhibitors (for example, L-NAME or L-NMDA), interferon, ketoprofen, ketorolac, leflunomide, mefenamic acid, mycophenolic acid, mizoribine, nabumetone, naproxen, oxaprozin, pyroxicam, rofecoxib, salsalate, sulindac and tolmetin, and corticoids such as cortisone, hydrocortisone, methylprednisolone, prednisone, prednisolone, betamethasone, betamethasone dipropionate, betamethasone valerate, beclomethasone dipropionate, budesonide, dexamethasone sodium phosphate, flunisolide, fluticasone propionate, paclitaxel, tacrolimus, tranilast, triamcinolone acetonide, fluocinolone acetonide, fluocinonide, desonide, desoximetasone, fluocinolone, triamcinolone, clobetasol propionate and dexamethasone. Ibuprofen is particularly suitable and preferred.

Use is preferably made of antithrombotics such as antiaggregants (acetylsalicylic acid, clopidogrel, ticlopidine, dipyridamole, abciximab, eptifibatide and tirofiban), anticoagulants (heparin, bivalirudin, dabigatran, lepirudin, fondaparinux, rivaroxaban, epoprostenol, warfarin, phenprocoumon, protein C, drotrecogin alfa, antithrombin, pentosan) and thrombolytics (alteplase, urokinase, tenecteplase and reteplase).

The use of a heparin is particularly preferred.

In another embodiment, ibuprofen is used.

In addition, it is possible to use a molecule which makes it possible to induce vascularization of the tissues surrounding the bioartificial organ, in particular PDGF (platelet derived growth factor), BMP (bone morphogenetic protein), VEGF (vascular endothelial growth factor), VPF (vascular permeability factor), EGF (epidermal growth factor), TGF (transforming growth factor) and FGF (fibroblast growth factor).

It is also possible to use IGF-1 and IGF-2, a neurotrophic factor (NGF).

In one particular embodiment, a cell growth factor is chosen which promotes vascularization by inducing angiogenesis, such as basic fibroblast growth factor (bFGF), vascular endothelial growth factor (VEGF), platelet derived endothelial cell growth factor (PDGF A or B), bone morphogenetic protein (BMP 2 or 4), or hepatocyte growth factor (HGF).

One can also use antimicrobial molecules, or any useful molecule such as polypeptides (in particular antimicrobial peptides), ionized silver, copper, zinc, non-metal elements such as hydrogen, chlorine, iodine, or oxygen, selenium.

For the preparation of the layer of hydrophilic polymer and biologically active molecule, the hydrophilic polymer or the blend of hydrophilic polymers is dissolved in water.

The addition of the hydrophilic polymer optionally containing an active molecule to the layer of porous biocompatible polymer is carried out according to the methods described in WO 02/060409 and WO 2012/017337.

In another embodiment, it is possible to add, at the surface of the porous biocompatible polymer, two layers each comprising a hydrophilic polymer and at least one biologically active molecule, as described in WO 2012/017337.

Reference is further made to EP86186, EP1112097, EP1112098, EP658112 and WO2012017337.

There are other ways to bind an active molecule to the surface of the membrane, illustrated with heparin below.

### Binding Heparin to a straight-chained organic polymer

In a specific embodiment, said heparin layer consists in a substantially straight-chained organic polymer having a number of functional groups distributed along the polymer backbone chain, via which groups at least 20 molecules of heparins are anchored through covalent bonds, wherein the heparins are bound to the polymer backbone chain *via* an amino group or amino acid associated with the heparins, and wherein said heparin layer is affinity bound to the surface of said layer of porous biocompatible polymer.

This method is actually disclosed in EP 658112.

Briefly, one uses an at least substantially water-soluble, biologically active conjugate (macromolecule), preferably in substantially pure form, comprising a substantially straight-chained organic homo- or heteropolymer having a number of functional groups distributed along the polymer backbone chain, via which groups of at least about 20 molecules of heparin are anchored through covalent bonds wherein heparin is bound to the polymer backbone chain via an amino group or amino acid associated with heparin. It could be noted that heparan sulphate, dermatan sulphate, chondroitin sulphate, but also fragments and derivatives of these substances which are functional for the purpose can be equivalently used and substituted to heparin. As indicated in EP 658112, the number of heparin residues per polymer backbone chain is, as mentioned above, at least 20, but preferably higher, usually at least 30. Depending on the polymer backbone chain used, it may be preferred to have at least 60 and even more than 100 heparin residues per polymer backbone chain. The upper limit depends on the circumstances and is set *inter alia* by the solubility properties of the selected carrier polymer, how high a viscosity that may be permitted, and the like.

### The substantially linear polymer

The substantially linear polymer chain is preferably substantially biologically inert after the coupling of heparin, i.e. devoid of interfering biological activity.

It should present a number of functional groups, such as amino, hydroxyl or carboxyl groups, distributed along the chain and capable of, after optional modification, being coupled to the heparin, either directly or via a coupling sequence.

The carrier polymer should preferably have a good solubility in water. At least it should, in accordance with what has previously been said about the conjugate, be at least substantially water-soluble after the coupling of heparin.

Specific and preferred polymer chains are natural or synthetic polypeptide, polysaccharide or aliphatic polymer, such as polylysine, polyornithine, chitosan, polyimine and polyallylamine.

### Binding of the conjugate polymer / heparin to the membrane surface

This conjugate is bound to the membrane surface, which may have been prepared to have affinity to the conjugate (usually but not necessarily to the heparin residues) so as to thereby provide the surface with the desired biological activity.

The functionalized membrane is obtained by simply contacting, under suitable conditions, the conjugate (comprising the organic polymer having a number of functional groups, via heparin molecules anchored by covalent bonds), with the membrane surface prepared to present affinity to the conjugate.

A preferred form of affinity between the conjugate and the substrate surface is of electrostatic nature, and more particularly that binding takes place by electrostatic interaction between the heparin residues and the membrane surface. Indeed, one will use the electrostatic net charge of the conjugate, which is sufficient to permit substantially irreversible binding to an oppositely charged membrane surface. Since the conjugate is negatively charged, the membrane surface shall be or shall be made cationic.

Various methods for making the membrane surface cationic are well known. Treatment with polyimine is a suitable method, but also other polyamines, such as polylysine, chitosan or polyallylamine, may be used. These polymers are used in the examples of EP 658112.

### Coupling of heparin to the polymer

There are different ways to couple the heparin to the substantially straight-chained organic polymer.

It is however preferred that each heparin molecule is bound terminally and by only a single bond to the carrier polymer. Suitably, the heparin molecule is bound via an amino acid, and then preferably a terminal amino acid, but also free amino groups of a heparin unit may be used. The latter may exist free as such or may have been liberated through desulphation or deacetylation.

In particular, the heparin may be bound directly to an amino-functional polymer chain utilizing a nitrous acid degraded heparin having a terminally located aldehyde group prepared according to the method described in US 4,613,665.

Preferably, the heparin is bound to the polymer chain by means of a coupling reagent, and preferably a heterobifunctional one, such as N-succinimidyl-3-(2-pyridyldithio)-propionate (SPDP).

As a way of illustration, coupling of heparin to a polylysine (having a molecular weight above 400,000) will be described to lead a conjugate having up to 500 heparin chains per carrier molecule may be prepared.

N-succinimidyl-3-(2-pyridyldithio)-propionate (SPDP), is coupled to amino groups on the polylysine, the SPDP-substituted polylysine then being purified chromatographically. In a separate coupling step, SPDP is also coupled to amino groups on heparin which are present either in terminal amino acid residues or as free glucosamine (the latter content may be controlled via N-desulphation or N-deacetylation). The SPDP-groups are reduced to thiol function, whereupon the SH-substituted heparin is purified chromatographically. The content of SPDP groups in polylysine and SH-groups in heparin, respectively, are determined spectrophotometrically, and heparin is mixed with polylysine in an equimolecular amount with regard to SPDP and SH, heparin being bound covalently to polylysine via disulphide exchange, the reaction rate of which may be followed spectrophotometrically. The precipitation reaction between polylysine and heparin does not take place when polylysine has been provided with SPDP-groups, even if only a certain portion of the amino groups of polylysine have been substituted. Practical experiments have shown that the disulphide exchange is quicker and proceeds to completion only at a high salt concentration (suitably 3 M NaCl). When the reaction is completed, the conjugate is purified chromatographically, free heparin and low-molecular reaction products being removed.

### Binding heparin by end-point attachment to the layer

In another embodiment, said heparin layer consists in heparin molecules covalently bound to a layer of a polymer applied on the surface of said layer of porous biocompatible polymer.

Methods to perform such embodiment are disclosed in details in EP 86186, EP 1112097 and EP 1112098.

The membrane surface is primed by applying a polymeric base matrix using a layer-by-layer technique. As a matter of illustration, the preferred method is to use a cationic amino polymer to be adsorbed to the material surface, followed by an anionic polymer and a polymeric amine. Additional layers of anionic and cationic polymers may also be applied to achieve optimal functional characteristics and coverage of the underlying material.

The technology is based on a chemical modification of heparin (diazotization, usually performed in an aqueous solution with a suitable diazotizing agent, e.g. a nitrite, such as sodium nitrite, in acid solution or butyl nitrite) that results in formation of a reactive aldehyde group at one end of the linear molecule. These groups are then reacted with primary amino groups incorporated on the material surface by the priming procedure, leading to formation of Schiff's bases, which are then reduced with a suitable reducing agent, such as a cyanoborohydride, preferably of an alkali metal, such as sodium, potassium or lithium, to yield stable covalent bonds.

### Physical characteristics of the biocompatible membrane

In the preferred embodiment, the membrane herein used to make the chamber of the kit comprises two layers of porous biocompatible polymer, each covered with at least one hydrophilic polymer, which surround the layer of non-woven.

### Pore diameter and density

As seen above, the pores are introduced into each of the layers of porous biocompatible polymer using methods known in the art (if the membrane comprises multiple such porous layers).

It is preferred when the pore density is greater than 10³ pores/cm², preferably greater than 10⁶ pores/cm². This pore density is generally less than 10¹¹ pores/cm², preferably less than 10¹⁰ pores/cm².

Use is therefore made of membranes which can have a pore density preferentially greater than 10⁶ pores/cm², more preferably greater than 10⁷ pores/cm². This density is preferentially less than 10¹¹ pores/cm², or even less than 10¹⁰ pores/cm². This density is therefore between 10³ pores/cm² and 10¹¹ pores/cm², preferably between 10⁶ pores/cm² and 10¹⁰ pores/cm². A density greater than 10⁷ and less than 10¹⁰ pores/cm² is perfectly suitable.

As seen above, the pores of the layers of porous biocompatible polymer have an internal diameter such that they allow semi-permeability of the membrane.

Thus, the cut-off of the membrane is obtained by pores which have an internal diameter greater than 5 nm and preferably greater than 10 nm, and less than 2000 nm, and preferably greater than 10 nm and less than 1200 nm, or the intervals indicated above. As indicated above, the purpose is to forbid entry of platelets within the chamber.

When the membrane has multiple layers of porous biocompatible polymers, both can present pores having the same internal diameter and the same pores density. Alternatively, the two membranes may present pores with different internal diameters, and/or different pores densities but which are in the ranges as described above. It is reminded that the cut-off is obtained by the smallest internal diameter of the pores present on each layer.

### Membrane thickness

In one preferred embodiment, the total thickness of the membrane (comprising the layer of non-woven polymer and the layer(s) of porous polymer(s)) is greater than 5 µm. It is generally, and preferably, less than 300 µm, but can also be greater than this size; thicknesses ranging up to 500 µm, or even beyond, can in particular be envisaged. Preferably, it is greater than 50 µm. It is also preferentially less than 250 µm. This membrane thus generally has a thickness of between 45 and 250 µm.

When the membrane has two layers of porous biocompatible polymers, said layers can have the same thickness or have different thicknesses.

The layer of non-woven polymer generally has a thickness greater than 20 µm, preferably greater than 60 µm, more preferably greater than 80 µm. This layer has a thickness generally less than 250 µm and preferably less than 150 µm. Thus, the thickness of the layer of non-woven polymer is often between 40 µm and 150 µm.

When the membrane has only one layer of biocompatible polymer, said layer then has a thickness greater than 5 µm. This layer is less than 200 µm, preferably less than 100 µm, being, however, preferably less than 50 µm.

When the membrane has two layers of porous biocompatible polymer, and said layers may have different thicknesses, the thickness of the one (preferably the internal, but it may be the external) layer is then greater than 5 µm. It is also preferably less than 200 µm, but preferably less than 50 µm; a thickness less than 15 µm (and preferably greater than 5 µm) is perfectly suitable. This thickness is preferentially the thickness of the layer which has pores with the smallest internal diameter, if the internal pore diameter is different for the two layers.

The thickness of the second layer can be in the same range as the one of the first layer, as indicated above. It can also be greater than 25 µm. It is preferably less than 200 µm, preferably less than 100 µm, more preferably less than 50 µm; a thickness of between 5 and 50 µm or 30 and 50 µm is perfectly suitable.

The thickness of each layer of hydrophilic polymer optionally present on one or the two layer(s) of porous biocompatible polymers is negligible, compared with the total thickness of the membrane. It is in fact preferably less than 500 nm and generally between 25 and 250 nm.

In one preferred embodiment, the membrane has two layers of porous biocompatible polymers on either side of a layer of non-woven polymer.

In this embodiment, one layer of porous biocompatible polymer has pores with an internal diameter greater than 100 nm, preferably greater than 200 nm, more preferably greater than 400 and less than 1200 nm, more preferably less than 700 nm, preferably at a density of about 5.10⁷ pores/cm². It is then advantageous for this layer to be the one with a thickness of between 25 and 200 µm (see above).

The size and density of the pores of the other layer of porous biocompatible polymer would be in the same range as for the first layer.

It is advantageous for this layer to have a thickness of between 5 and 200 µm (preferably 5 to 50 µm).

The ranges of the size, density of the pores, and the thickness of the layers, when the chamber comprises two layers of porous biocompatible polymer are generally the same: pores size: between 5 nm and 2000 nm ; thickness: between 5 and 50 microns ; density between 10⁶ and 10¹⁰ pores/cm².

### Connector

US 2013216746 describes connectors that can be used with the chamber present in the kit. As for this patent application, a connector usable in the present kit may comprise a cap, a body and a base. The cap would comprise a central cavity connected to a sleeve, which receives the catheter, which is, for example, bonded to the inside of the sleeve. The body may have an annular shape and comprises three body teats projecting toward the cap. The cap may comprise three holes opposite the body teats so as to produce an assembly between the body and the cap by fitting the body teats into the holes. The body may also comprise an annular bulge corresponding to a recess of complementary shape produced in the cap, so as to clamp and hold one of the membranes forming the chamber, called an upper membrane, between the cap and the body.

### Catheter

The catheter used in the kit is any catheter known in the art, and is made in a biocompatible material. It is a flexible catheter which can be connected, at one end, to the connector on the surface of the chamber, so that the catheter's lumen is in relation with the interior volume of the chamber. The other end catheter may be connected to an injection port, a pump, or a vessel containing the compound of interest.

The internal diameter is generally in the range of 1 mm (i.e. it is generally between 0.5 mm and 1.5 mm, more preferably between 0.8 mm and 1.2 mm, or 0.9 mm and 1.1 mm). However, catheter of larger diameters may also be used.

The catheter is made in any biocompatible material such as silicone or any other biocompatible elastomer.

### Injection Port

Injection ports are known in the art. In the context of the present application, an injection port is a port that is to be placed near the surface of the skin of the patient (in a preferred embodiment, it is implanted subcutaneously).

In the context of the invention, an injection port may present a small chamber (portal, housing that defines a reservoir) with a septum disposed across an opening of the housing so as to cover the reservoir, wherein at least a part of this septum is penetrable by a needle. The septum is generally a rubber one, or can be made in silicon.

A needle can be used to inject the solution containing the compound of interest in the injection port, through the rubber septum. Once injected the solution will transitorily pass through the housing and flow to the implanted chamber by the catheter, where it will diffuse *in situ* through the pores of the membrane.

Implanting such an injection port makes it possible for the patient or the physician to easily inject the compound of interest while reducing the risks of infection and eliminating the pain associated with injections.

Multiple injection ports exist and have been described in the art, such as the the injection ports of the Districath® series (Districlass Medical SA, Saint-Etienne, France), the injection ports developed by B-Braun (Melsungen, Germany) or in US 20110184353.

One can use any other injection port such as the injection ports that are used in chemotherapy.

Because it is preferred when the port is implanted under the skin, it may be accessed using a dedicated needle or by connecting a pump through a catheter. The skin would be cleaned with an antiseptic and the needle inserted through the skin and the rubber septum. Ports can be implanted sub-cutaneous, on any appropriate location such as on the abdomen, but also on other areas such as the buttocks, thigh or arm.

The ports are generally made of titanium (or any thermoplastic such as Polysulfone) for their body (housing) and of silicone (septum).

The existing subcutaneous injection ports make it possible to perform multiple injections over the time. In particular, using the appropriate injection system, it may be possible to perform about 1000 injections of the compound of interest. For a patient with diabetes, which would make about 5 injections a day, this means that the ports could remain in place for about 200 days (8 months). This would ensure good healing, and prove to be an improvement in the life of patients who currently need to change the system every third days with external insulin pumps.

One can use any appropriate injection system such as a needle which would preferably be between 4 and 35 mm long, more preferably between 5 and 25 mm, most preferably between 8 and 13 mm. The diameter of the needle would preferably be between 18 and 32 gauges, most preferably between 22 and 32 gauges.

### Sensors

The kit herein described may also contain one or multiple sensors for monitoring glucose concentration in the blood of the host, and to provide measured data associated with the glucose concentration. An electronics module can also be part of the kit that will determine the insulin quantity to provide to the patient and send instructions to an insulin delivery device configured to deliver insulin to the host.

Such sensors are known in the art and are described in various publications such as US 9,463,277, US 9,457,146, US 9,452,260, US 9,452,259, US 9,452,258 or 9,283,323. This sensor is generally a continuous glucose monitoring (CGM), which will determine glucose levels at regular intervals (in the range of 2 to 5 minutes).

### Implantation of the chamber

The chamber of the kit is implanted at the most appropriate site to have a delivery of the compound of interest at a site of physiological interest.

As a matter of illustration, the chamber is implanted between muscles (abdominal muscles or rectus abdominis muscles) and the peritoneum when the compound of interest is insulin, for treating diabetes, in particular type 1 diabetes mellitus, or type 2 diabetes.

The chamber may be implanted at any site of interest, depending of the compound of interest to be delivered and the expected biodistribution. It can be implanted, in particular, sub-cutaneously, extraperitoneally, or intraperitoneally. The examples describe a specific procedure for extraperitoneal implantation.

### Compound of interest

The compound of interest is any compound that is injected to a patient for treating the patient.

it can be, in particular, a growth hormone (for treating dwarfism), a coagulation factor (for treating hemophilia), a cytokine or the like (tumor-necrosis factors, interferons...) or an anti-inflammatory molecule (whether nonsteroidal or steroidal) useful for treating auto-immune diseases such as arthritis, ankylosing spondylitis, multiple sclerosis, celiac disease, Graves disease, inflammatory bowel disease, psoriasis, rheumatoid arthritis, and systemic lupus erythematosus, heparin or heparinoids useful for treating coagulation, a compound used in immunotherapy, a drug used in chemotherapy, an immunosuppressing drug (such as for treating Graft vs Host rejections), an antiviral drug, arsenic (some auto-immune diseases), TNF (useful for hepatitis C), dopamine (for Parkinson disease treatment), eptifibatide (for reducing the risk of acute cardiac ischemic events and treating heart failure), a beta blocker drug.

One advantage of the kit herein disclosed is that it allows a diffusion of the compound of interest, an improved biodisponibility, and the possibility to easily ensure either continuous or discrete (but repeatable) administration of the compound of interest.

### Use of the kit

The kit as described herein may be used on human beings as well as on animals, in a veterinary usage, such as for insulin administration for dogs and cats.

### Some Advantages of the kit for treatment of Diabetes

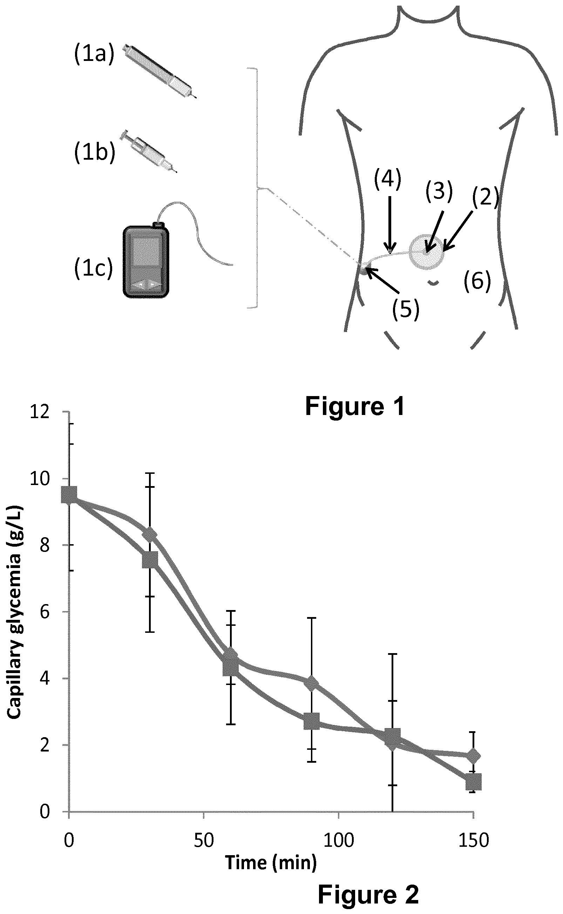

- Most insulin administered via the extraperitoneal or intraperitoneal route rapidly reaches the liver via the portal vein, more quickly than in the case of a subcutaneous administration. Insulin diffusion in vivo is similar to direct intraperitoneal injection (see figure 2).
- The method should lead to an improved regulation of blood glucose by reducing the frequency of severe hypoglycaemia
- Using the kit would lead to an improved quality of life for diabetic patients
- A diffuse repartition of insulin, which will reduce or avoid any focal steatoses linked to local hyperinsulinism, and insulin-resistance
- No risk of infection (in comparison to Diaport® system from Roche) since the system and the implantation procedure are safe
- No adhesion if extraperitoneal implantation, as compared with other devices implanted intraperitoneally (such as Diaport®) which may adhere to the tissues. This is particularly true if the surface of the chamber is coated with anti-inflammatory and/or anti-adhesion molecules.
- No risk of occlusion of catheters (in comparison to Diaport® system from Roche) by surrounding tissue, since the catheter is connected to the interior of the chamber.
- Long-time implantation when a sub-cutaneous injection port is used, when there is a need to change the catheter every three days for current insulin pumps.

### Description of the figures

Figure 1: Representation of a fit for insulin/drug intraperitoneal/extraperitoneal delivery, composed of an external (or implanted) drug delivery system (1), which can be a pen (1a), syringe (1b), injector, external or internal (implanted) pump (1c) or artificial pancreas, linked to an implantable pocket (2), made with a biocompatible semi-permeable membrane sealed at its periphery, *via* a connection system comprising connectors (3). The kit comprises the implantable chamber (2), at least one catheter (4) and at least one connector (3) for connecting the lumen of the catheter (4) to the interior of the pocket (2), thus linking the external drug injection system (1) and the pocket (2). The compound from the drug delivery system (1) is injected in the catheter (4) through injection ports (5) generally implanted subcutaneously. The pocket / chamber (2) is implanted where delivery of the compound is desired (6), such as intraperitoneally or extraperitoneally.
Figure 2: Plasmatic glycaemia of diabetic rats which received insulin with a kit as herein described, (circles) or directly via intra-peritoneal injection (squares). Results are presented as mean ± standard deviation.

### Examples

### Example 1 - Use of a chamber in rats

Rapid Insulin was injected through a catheter linked to a pocket as disclosed in the examples of WO 2015/086550 (three layer, including one of non-woven polymer). The pocket was implanted in the intraperitoneal cavity of rats.

The animals were diabetic rats and injection of insulin induced a rapid decrease of plasmatic glycaemia (Figure 2, circles).

The kinetics of action are similar as the ones observed with a direct intraperitoneal injection of insulin (Figure 2, squares), indicating an instant diffusion of insulin out of the device.

### Example 2 - Extraperitoneal implantation procedure

### Preparation

Directly before the surgical procedure, an intravenous (IV) line is placed so that fluids and/or medications may be administered to the patient during and after surgery.

### Anesthesia

The patient is usually placed under general anesthesia for the duration of surgery.

### Preparation

Lie the patient on his back. Shave the patient locally and use antiseptic solutions to clean the skin shaved. Drape the patient's abdomen and fix the drapes with towel clips. Cover these with a large windowed sheet, and add additional sterile towels as necessary. When the patient is anaesthetized and relaxed, feel his abdomen carefully by palpation.

### Incision

A scalpel is first used to cut into the superficial layers of the skin.

Make a midline incision which is big enough to allow you to insert the chamber and get at the organs you want to operate on.

The incision is realized through skin, subcutaneous fat and *linea alba* until the peritoneum is exposed. To avoid accidental perforations of the peritoneum by cutting the *linea alba* the incision is slightly deported from the medina plan.

Incise 1 to 2 mm from the median plan using a scalpel or an electric bistoury. Cut down to the *linea alba* and then carefully dissect the fat for a centimeter on either side, using the flat of the knife.Then incise the *linea alba* to expose the underlying fat and peritoneum. Displace the fat and vessels laterally.

### Chamber implantation

### Pocket preparation

A pocket has to be designed for the implantation of the chamber, between the retromuscular and properitoneal space (between abdominal transverse muscle *[transversus abdominis]* and the peritoneum).

Dissect gently the abdominal muscles from the peritoneum using fingers or the flat of the scissors until to be under the abdominal transverse muscle and make sure that the pocket is large enough to receive the chamber

### Chamber Placement

Prior to insert the chamber, permeate it using sterile gauze and sterile saline solution. Insert the chamber in the implantation pocket site previously made. Make sure that the top part of the chamber which includes catheter is placed to be in contact with abdominal transverse muscle. Make that catheters have proximal midline incision orientation.

### Chamber Suture

The chamber could be sutured to the fascia by using 2/0 or 3/0 braided non absorbable filament.

### Implantation Site Closure

The organs and related structures are returned to their normal anatomical position. The layers of the abdominal wall are sutured in reverse order using braided absorbable suture (1 or 2).

### Implanted Ports Placement

### Catheter Tunneling

Create subcutaneous pockets using a blunt dissection. Make sure that pocket is large enough to accommodate the port so that the scar does not overlap it. Attach the distal part of the catheter onto the tunneler or the Redon'awl barb with a twisting motion. Make sure that the barb threads are completely covered by the catheter to secure the catheter as it is pulled through the tunnel (a suture may be tied around the catheter and the barb).

Pull the tunneler through the port pocket site while holding the catheter.

Cut the catheter to the proper length at 90° angle, allowing sufficient slack for the body movement (avoiding tensile strength) and port connection.

### Catheter Connection to Port

Prior to connecting the port with the catheter, ensure that the catheter is properly positioned to fit with the port pocket site. Insert the catheter lock (titanium ring) on the tunneled catheter. The catheter should be straight and have no sign of kinking. Connect the catheter to the port. Insert the catheter on the port (about) 5 mm. Hold the catheter and the catheter lock together and push the catheter lock until it reaches the port.

### Position and Closure

Place the port in the subcutaneous pocket away from the incision line. Stitch the port with non-absorbable monofilament sutures to the underlying fascia using the perforations on the port. This will reduce the risk of port migration and/or it flip over. Leave enough slack in the catheter to permit slight movement and verify that the catheter is not kinked. After suturing the port in the pocket, flush the pocket with appropriate solution (antibiotics or iodine solution). Close the incision site with absorbable 2/0 or 3/0 braided suture.

### Skin Closure

Upon completion of the procedure, the incision may then be sutured (stitched closed). The skin incision is closed with sutures using 2/0 or 3/0 braided absorbable filament.

## Claims

1. A kit comprising
a) a chamber comprising a closed shell made of the semi-permeable membrane, wherein the pouch comprises at least one connector comprising a body attached to the sheet, and a conduit connected to the connector so as to be in hydraulic communication with the inside of the pouch
b) a catheter that can be connected to said connector at one end and that can be connected to a source of delivery of a compound of interest at the other end.

2. The kit of claim 1, wherein said catheter presents an injection port, in particular implantable subcutaneously, at the end that can be connected to a source of delivery.

3. The kit of claim 1 or claim 2, which further comprises a vessel containing said compound of interest.

4. The kit of claim 3, which further comprises a pump, a needle or a pen, making it possible to send the compound of interest from the vessel to the chamber within the catheter.

5. The kit of any one of claims 1 to 4, wherein the internal diameter of membranes pores (semi-permeability) is comprised between 5 nm and 2000 nm, preferably between 10 nm and 1200 nm.

6. The kit of any one of claims 1 to 5, wherein said the membrane of the chamber comprises multiple layers of biocompatible polymers.

7. The kit of claim 6, wherein said semi-permeable membrane comprises at least one layer of porous biocompatible polymer, and one layer of non-woven biocompatible polymer.

8. The kit of any one of claims 1 to 7, wherein the membrane comprises a biologically active molecule on its external and/or internal surface.

9. The kit of claim 8, wherein said molecule is covalently bound to a layer on the surface of said membrane.

10. The kit of any one of claims 1 to 9, further comprising sensors and/or captors to measure the level of a given biomarker in the blood and optionally send signals to the external source of the compound of interest.

11. The kit of claim 10, wherein said biomarker is glucose and wherein said compound of interest is insulin.

12. The kit of claim 1, wherein said source of delivery is a syringe, an artificial organ, an implantable or external vessel optionally also comprising a pump, a needle or a pen.

13. A method for delivering a compound of interest to a patient comprising the steps of using the kit according to any one of claims 1 to 12 in order to deliver the compound of interest to the interior of the chamber, from the external source, through the catheter.

14. The method according to claim 13, wherein the chamber has previously been implanted between the muscles (rectus or Transversus abdominis muscles) and the peritoneum.

15. A method of using the kit of any one of claims 1 to 12, comprising the step of surgically implanting the chamber between the muscles (rectus or Transversus abdominis muscles) and the peritoneum.
